# EUROPEAN PATENT SPECIFICATION

(11) **EP 2 014 258 B1**
(45) Date of publication and mention of the grant of the patent: **06.04.2011**
(21) Application number: 07112176.8
(22) Date of filing: 10.07.2007
(51) Int. Cl.: A61F 2/28, A61F 2/30, A61B 17/80, A61B 17/68

(54) **Cranial implant**
Schädelimplantat
Implant crânien

(43) Date of publication of application: 14.01.2009
(73) Proprietor: Maastricht Instruments B.V., 6229 ER Maastricht (NL)
(72) Inventor: Nijenhuis, Gerard, 2613 GL, Delft (NL); Laeven, Paul Frans Jozef, 6294 NL, Vijlen (NL); Poukens, Jules Maria Nikolaas, B-3650, Elen-Dilsen (BE)
(74) Representative: Aalbers, Arnt Reinier

(56) References cited:
- EP-A- 1 099 416
- US-A1- 2002 022 844
- US-A1- 2006 224 242
- US-B1- 6 197 037

## Description

The invention relates to an implant, such as a cranial implant, comprising a rim and one or more fasteners for attaching the rim to an edge of a defect in a bone structure.

Implants are employed for repairing defects in bone structures resulting from surgery or trauma. An implant closes a defect, such an opening in a skull, protects the tissue underneath, e.g. brain tissue, and/or restores the contour of the bone structure.

Implants may have different origins. For instance, autogenous grafts are taken from one part of a human or animal body and implanted in another part of that same body and allografts are bone grafts taken from an individual from a species and inserted in the body of another individual of that same species. Alloplastic implants are implants made from body foreign material.

All implants have in common that they must be securely fixed to the bone structure. Implant migration and micro-movements may hinder osteointegration (sometimes also referred to as osseointegration) and even result in rejection of the implant.

In US 2006/0116682, a biocompatible and pliable surgical implant for use in repair and rigid fixation of bone fractures and to compensate for a volume loss is disclosed. The implant includes a biocompatible base member (plate, strip or panel) and a volume member having a predetermined volume affixed to the metallic base member. The base is adapted to be secured proximate the fractured bone area. In the examples, the base comprises one or more extensions or fingers having a suitable through-opening or hole for inserting a securing member, such as a bone screw, therethrough.

US 6,618,623 discloses a ferrule for retaining an implantable device within a cranial opening of a patient. The ferrule is inserted into the cranial opening and fixed to the cranium. Then an implantable device is inserted into the ferrule and secured thereto. In the examples, the ferrule is secured to the patient's cranium with (self-tapping) bone screws.

US 6,197,037 discloses a surgical fastener for joining adjacent bone portions includes a top flange and a bottom flange joined by a rib that together help define two bone receiving cavities. The Respective bone edge portions, such as from an osteotomy cut, are inserted into the bone receiving cavities and gripped by the compressive action between the top flange and the bottom flange. In the installed state, the top flange is disposed on one side of the bone while the bottom flange is disposed on the opposite side of the bone, with the rib disposed between the bone edges.

US 2006/224242 discloses a composite surgical implant that is made of a planar sheet of a thermoplastic resin that includes a top surface, a bottom surface, and a surgical grade metal mesh or metal plates contained therein.

US 2002/022844 proposes an implant designed as a U-shaped clip, with a bridge and two contact arms protruding transversely from the bridge, the contact arms being capable of receiving a bone plate between them and thereby fixing the clip on the bone plate with the contact arms lying against the upper side and the lower side, respectively, of the bone plate, and with at least one projection on the outer side of the bridge facing away from the contact arms for fixing the clip on the other bone plate, so that a displacement of the clip relative to the other bone plate in the longitudinal direction of the bridge is prevented in at least one direction.

Bone screws, tacks and the like necessitate further damage to the bone structure and, once applied, exert (lateral) compressive forces on the bone structure. Compressive forces in turn may cause resorption of bone and loosening of the screws.

It is an object of the present invention to provide an improved implant.

To this end, the implant according to claim 1 is provided.

In another aspect of the invention, the extendible element is formed on or by one end of a clip and the other end of the clip comprises a feature, such as two diverging resilient legs, for releasably fixing the clip relative to at least the rim. The clip can comprise or consist of a shaped wire.

In a further aspect of the invention, the at least one element is resilient, the fastener(s) further comprise(s) a guide for the resilient element, and the resilient element is mounted in the guide.

The present implant does not require the drilling of holes or other forms of damage to the bone structure. Further, in at least some of the embodiments, the fixing of the implant is reversible, i.e. the implant can be removed if complications, such as bleeding, occur or if it appears that adjustments regarding the position or shape of the implant are desirable.

The invention will now be explained in more detail with reference to the figures, which show several embodiments and details of the present implant.
Figure 1 is a perspective view of part of a skull provided with an implant according to the present invention.
Figure 2 is a cross-section of the skull shown in Figure 1.
Figures 3 and 4 are perspective views of the components of a embodiment of the fastening means used in the implant according to Figures 1 and 2.
Figures 5A to 5F are perspective, cross-sectional, top, front, side, and rear views, respectively, of a second embodiment of fastening means.
Figure 5G is a perspective view of the entrails of the fastening means shown in Figures 5A to 5F.
Figure 6 is a cross-section of the skull provided with an implant comprising the fastening means of Figures 5A to 5G.
Figures 7A to 7F are perspective, cross-sectional, top, front, side, and rear views, respectively, of a third embodiment of fastening means.
Figure 8 is a cross-section of the skull provided with an implant comprising the fastening means of Figures 5A to 5F.

It is noted that the drawings are not necessarily to scale and that details that are not required for understanding the present invention may have been omitted. The terms "upward", "downward", "below", "above", and the like relate to the embodiments as oriented in the drawings. Further, elements that are at least substantially identical or that perform an at least substantially identical function are denoted by the same numeral.

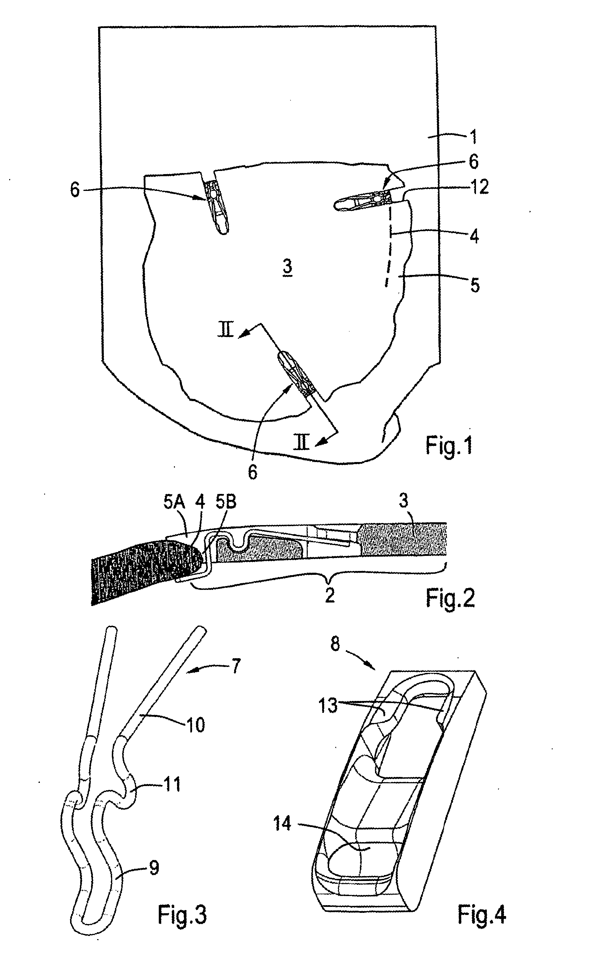

Figure 1 shows a human skull 1 having a defect 2 resulting from surgery or trauma, which defect 2 is closed by means of a first embodiment of a cranial implant 3 according to the present invention. As shown in Figure 2, the defect 2 has a rounded edge 4 indicating that the external and internal laminae cover the diploe and thus that the wound has been allowed to heal, typically for two to six months.

In this example, the implant is made of a metal, for instance titanium grade 5. Alternative materials include, but are not limited to, polymers, e.g. polymethyl methacrylate (PMMA), polyether ether ketone (PEEK), or polyether ketone ketone (PEKK), and ceramic materials, e.g. zirconium oxide or aluminum oxide. The rim 5 of the implant 3 can be closely matched to the shape of the edge of the defect by means of CT-scans and 3D modelling, in a manner known in itself.

The side 5A of the rim 5 of the implant 3 facing outwards, i.e. the side that is at least substantially flush with the "lamina externa" of the skull 1, is broader than the side 5B of the rim facing inwards, which rim 5B is at least substantially flush with the "lamina interna". Upon placing the implant 3 in the defect 2 in the skull 1, the rim 5A rests on the edge 4 of the defect 2 and, in view of the close fit of the rim 5 and the edge 4, only one degree of freedom remains, i.e. in a direction opposite to the direction of placing the implant 3. To fix the implant 3 in this remaining direction, the first embodiment of the present implant 3 comprises, along its rim 5, a plurality, e.g. three fasteners 6. Each of the fasteners 6 comprises a resilient clip 7 and a clip mount 8.

The clip 7 comprises on one of its ends an extension 9, which is to be fitted under the bone structure 1. The remainder of the clip 7 comprises features for releasably fixing the clip 7 relative to at least the rim 5. In this example, these features include two diverging resilient legs 10 on the other end of the clip 7 and two U-bends 11 located in between the ends and directed downwards. The clip 7 is made of a metal wire. Suitable metals include titanium grade 5.

The clip mount 8, which can be an integral part of the rim 5 of the implant 3 or a separate element that is fixed to at least the rim, provides a first recess 12 (shown in Figure 1) in the outer rim 5 of the implant 3 and comprises, near its end remote from the outer rim, two protrusions 13 and, in between its ends, a further recess 14.

The implant 3 can be secured in the defect 2 by placing each of the clips 7 in its respective clip mount 8, as follows:
- urging the legs 10 of the clip 7 together,
- guiding the extension 9 of the clip 7 through the first recess 12,
- turning the legs 10 towards the implant 3, thus turning the extension 9 along the surface of the edge of the bone structure 1 and positioning the U-bends 11 in the further recess 14,
- moving the legs 11 past the protrusions 13, and
- releasing the legs 10.

After inserting all the clips 7, the extensions 9 resiliently press against the inner surface of the skull 1. The implant 3 can be removed if complications, such as bleeding, occur or if it appears that adjustments to the implant 3 are desirable. In most patients, osteointegration will provide secure fixing of the implant 3 to the skull 1 after some weeks, typically after six to eight weeks. The resilience of the extensions 9 will compensate for local reduction of the thickness of the bone structure 1 resulting from resorption.

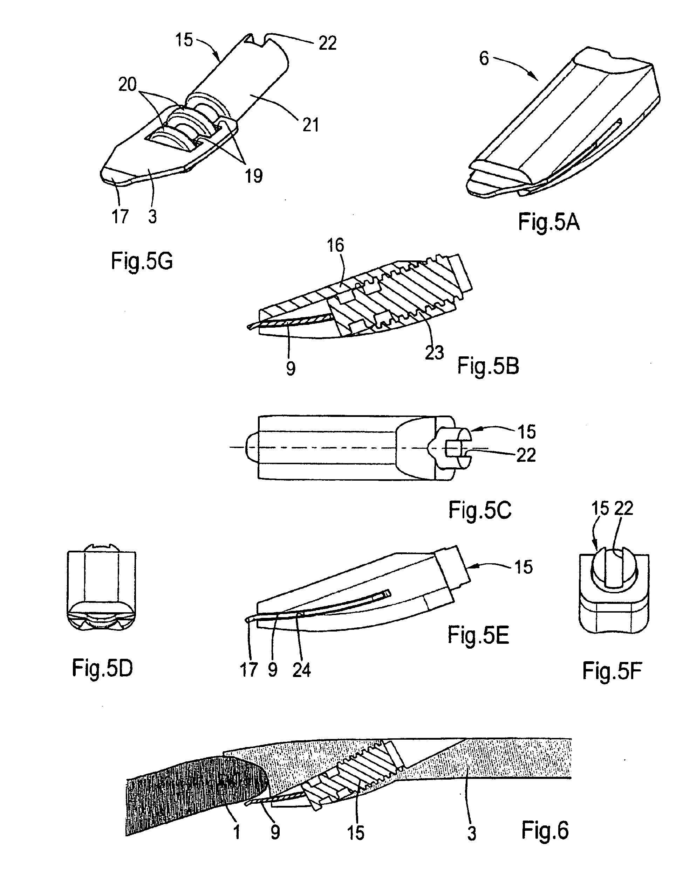

Figures 5A to 6 show a second embodiment of a fastener 6. This fastener 6 comprises a resilient blade 9, an adjuster 15 for the blade 9, and a guide 16. The distal end 17 of the blade 9 is slightly curved downwards to prevent it from causing damage to the inner surface of the skull 1. At its proximal end, the blade 9 comprises an opening, in this case comprising two rectangular cutouts 19 (Figure 5G), for coupling the blade 9 to the adjuster 15. The adjuster 15 comprises on its distal end a rotationally symmetric feature, e.g. one or more discs 20, fitted in the opening 19 in the blade 9. An externally threaded portion 21 is provided near the proximal end of the adjuster 15. The proximal end itself comprises a feature, e.g. a transverse slot 22, for temporarily coupling the adjuster 15 with a driver, such as a surgical screw driver. In this example, the blade 9 and the adjuster 15 are made from titanium grade 5.

The guide 16 comprises a corresponding internally threaded portion 23, such that by rotating the adjuster 15 clockwise or counter-clockwise, it moves outwards or inwards thus extending or retracting the blade 9. The guide 16 further comprises a curved bottom portion or slot 24, which urges the distal end 17 of the blade 9 upwards. The guide 16 can be an integral part of the rim 5 of the implant 3, as shown in Figure 6, or a separate element that is fixed to at least the rim. Upon placing the implant 3 in a defect 2, the implant 3 can be secured to the bone structure 1 by rotating the adjuster(s) 15 thus extending the blade(s).

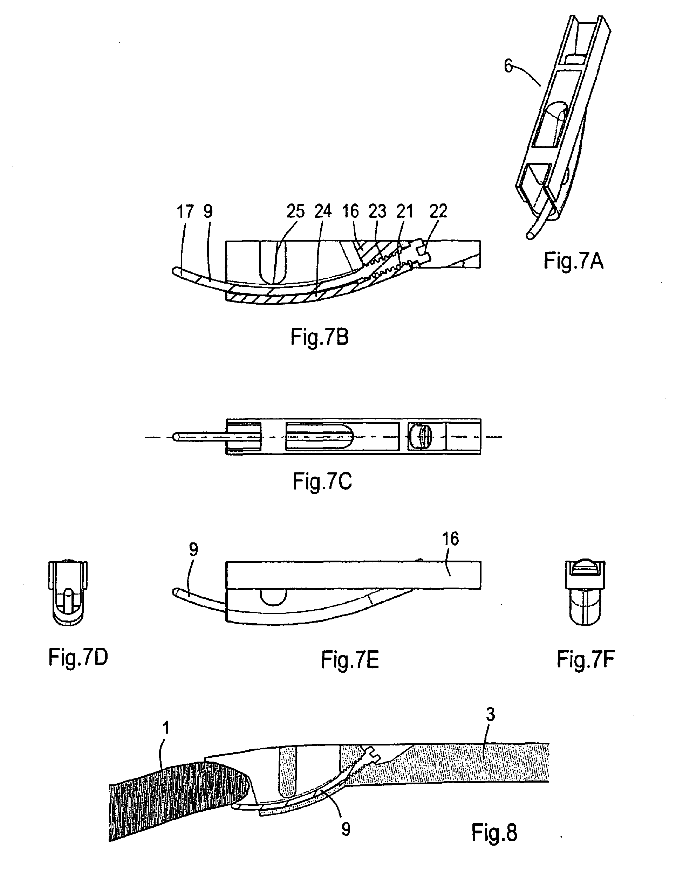

Figures 7A to 8 show a third embodiment of a fastener 6. This fastener 6 comprises a resilient pin 9 and a guide 16. The distal end 17 of the pin 9 is rounded, whereas the proximal end comprises a feature, e.g. a transverse slot 21, for temporarily coupling the pin 9 with a driver, such as a surgical screw driver. An externally threaded portion 21 is provided near the proximal end of the pin 9. In this example, the pin is made of PMMA. Other suitable materials include, but are not limited to poly lactide polylactic acid (PLA), poly L-lactic acid (PLLA), and poly D-lactic acid (PDLA).

The guide 16 comprises a corresponding internally threaded portion 23, such that by rotating the pin clockwise or counter-clockwise, its distal end is extended or retracted. The guide 16 further comprises a curved bottom portion 24, which bends the pin 9 and urges the distal end 17 of the pin 9, upon placing the implant 3 in the defect 2, towards the bone structure 1, and a cross-bar 25, which prevents the pin 9 from slipping out of the guide 16. The guide 16 can be an integral part of the rim 5 of the implant 3, as shown in Figure 8, or a separate element that is fixed to at least the rim. Upon placing the implant 3 in a defect 2, the implant 3 can be secured to the bone structure by rotating the pin(s) 9.

The embodiments described above do not require the drilling of holes or other forms of damage to the bone structure. Further, these embodiments comprise few components and the securing of the implant in a defect is relatively straightforward and reversible. Once secured, micro-movements of the implant relative to the bone structure are prevented or at least reduced, thus avoiding or at least reducing the risk of the body rejecting the implant.

The invention is not restricted to the above-described embodiments, which can be varied in a number of ways within the scope of the claims. For instance, instead of threaded portions other means for extending and securing the blade or pin described above can be provided, such as releasable click fingers.

## Claims

1. Implant, such as a cranial implant (3), comprising a rim (5) and one or more fasteners (6) for attaching the rim (5) to an edge (4) of a defect (2) in a bone structure (1), wherein one side (5A) of the rim (5) is broader than the other side (5B) of the rim (5) and/or comprises one or more protrusions extending beyond the periphery of the other side (5B) of the rim (5), such that, upon placing the implant (3) in the defect (2), the rim (b) and/or protrusions rest/s on the edge (4) of the defect (2), wherein the fastener/s (6) is/are mounted or mountable in or on at least the rim (5) and comprise/s at least one element (9) which is extendible in a direction away from the rim (5)and under the bone structure (1), **characterized in that** the implant comprises at least one guide (12, 16) and **in that** the extendible element (9) is extendible through said guide (12, 16).

2. Implant (3) according to claim 1, wherein the element (9) is formed on or by one end of a clip (7) and the other end of the clip (7) comprises a feature, such as two diverging resilient legs (10), for releasably fixing the clip (7) relative to at least the rim (5).

3. Implant (3) according to claim 2, wherein the clip (7) comprises or consists of a shaped wire.

4. Implant (3) according to any one of the preceding claims, wherein the at least one element (9) is resilient and wherein the fastener/s (6) further comprise(s) a guide (16) for the resilient element (9) and the resilient element (9) is mounted in the guide (16).

5. Implant (3) according to claim 4, wherein the resilient element (9) comprises a threaded portion (21) or is coupled to a further element (15) comprising a threaded portion (21) and is mounted in the guide (16) by means of at least this threaded portion (21), such that the resilient element (9) can be extended or retracted by rotating this portion (21).

6. Implant (3) according to claim 4 or 5, wherein the guide (16) is at least partially curved and urges the resilient element (9) towards the bone structure (1).

7. Implant (3) according to any one of the preceding claims, wherein at least the shape of the rim (5) of the implant is modeled to closely match the shape of the edge (4) of the defect (2), such that, upon placing the implant (3) in the defect (2), only one degree of freedom remains.

8. Implant (3) according to any one of the preceding claims, wherein the extendible element (9) is sufficiently resilient to compensate for local reduction of the thickness of the bone (1).

## Patentansprüche

1. Implantat, zum Beispiel ein Kranialimplantat (3), aufweisend einen Randbereich (5) und ein oder mehr Befestigungselemente (6) zum Anbringen des Randbereiches (5) an einem Rand (4) eines Defekts (2) in einer Knochenstruktur (1), wobei eine Seite (5A) des Randbereiches (5) breiter als die andere Seite (5B) des Randbereiches (5) ist und/oder einen oder mehr Vorsprünge aufweist, der/die sich über den Umfang der anderen Seite (5B) des Randbereiches (5) hinaus erstreckt/erstrecken, so dass der Randbereich (5) und/oder die Vorsprünge auf dem Rand (4) des Defektes (2) beim Platzieren des Implantates (3) in dem Defekt (2) aufliegt/aufliegen, wobei das Befestigungselement/die Befestigungselemente (6) in oder an zumindest dem Randbereich (5) montiert oder montierbar ist/sind und mindestens ein Element (9) aufweist/aufweisen, das in einer Richtung von dem Randbereich (5) weg und unter die Knochenstruktur (1) erstreckbar ist, **dadurch gekennzeichnet, dass** das Implantat mindestens eine Führung (12, 16) aufweist und dass das erstreckbare Element (9) durch die Führung (12, 16) hindurch erstreckbar ist.

2. Implantat (3) gemäß Anspruch 1, wobei das Element (9) an einem oder durch ein Ende eines Clips (7) ausgebildet ist und das andere Ende des Clips (7) ein Merkmal, wie zum Beispiel zwei divergierende federnde Schenkel (10) zum lösbaren Befestigen des Clips (7) in Bezug zu mindestens dem Randbereich (5) aufweist.

3. Implantat (3) gemäß Anspruch 2, wobei der Clip (7) einen Formdraht aufweist oder aus einem solchen gebildet ist.

4. Implantat (3) gemäß einem der vorhergehenden Ansprüche, wobei das mindestens eine Element (9) federnd ist und wobei das Befestigungselement/die Befestigungselemente (6) ferner eine Führung (16) für das federnde Element (9) aufweist/aufweisen, und das federnde Element (9) in der Führung (16) montiert ist.

5. Implantat (3) gemäß Anspruch 4, wobei das federnde Element (9) einen Gewindeabschnitt (21) aufweist oder an ein weiteres Element (15) angeschlossen ist, das einen Gewindeabschnitt (21) aufweist, und mittels mindestens des Gewindeabschnitts (21) in der Führung (16) montiert ist, so dass das federnde Element (9) durch Drehen des Abschnitts (21) erstreckt oder zurückgezogen werden kann.

6. Implantat (3) gemäß Anspruch 4 oder 5, wobei
die Führung (16) zumindest teilweise gekrümmt ist und das federnde Element (9) in Richtung zu der Knochenstruktur (1) zwingt.

7. Implantat (3) gemäß einem der vorhergehenden Ansprüche, wobei mindestens die Form des Randbereiches (5) des Implantats derart modelliert ist, dass sie eng zu der Form des Randes (4) des Defektes (2) passt, so dass beim Platzieren des Implantats (3) in dem Defekt (2) nur ein Freiheitsgrad verbleibt.

8. Implantat (3) gemäß einem der vorhergehenden Ansprüche, wobei das erstreckbare Element (9) ausreichend federnd ist, um eine lokale Verringerung der Dicke des Knochens (1) zu kompensieren.

## Revendications

1. Implant, tel qu'un implant crânien (3), comprenant une collerette (5) et une ou plusieurs fixations (6) pour fixer la collerette (5) à un bord (4) d'un manque (2) dans une structure osseuse (1), dans lequel un côté (5A) de la collerette (5) est plus large que l'autre côté (5B) de la collerette (5) et/ou comprend une ou plusieurs saillies s'étendant au-delà de la périphérie de l'autre côté (5B) de la collerette (5), de sorte que, lors de la mise en place de l'implant (3) dans le manque (2), la collerette (5) et/ou les saillies s'appuie(nt) sur le bord (4) du manque (2), dans lequel la (les) fixation(s) (6) est/sont montée(s) ou peut (peuvent) être montée(s) dans ou sur au moins la collerette (5) et comprend (comprennent) au moins un élément (9) qui est extensible dans un sens s'éloignant de la collerette (5) et sous la structure osseuse (1), **caractérisé en ce que** l'implant comprend au moins un guide (12, 16) et **en ce que** l'élément extensible (9) est extensible à travers ledit guide (12, 16).

2. Implant (3) selon la revendication 1, dans lequel l'élément (9) est ménagé sur ou par une extrémité d'un clip (7) et l'autre extrémité du clip (7) comprend une caractéristique, comme deux pattes résilientes divergentes (10), pour fixer de manière amovible le clip (7) relativement à au moins la collerette (5).

3. Implant (3) selon la revendication 2, dans lequel le clip (7) comprend ou est constitué d'un fil façonné.

4. Implant (3) selon l'une quelconque des revendications précédentes, dans lequel ledit au moins un élément (9) est résilient et dans lequel la (les) fixation(s) (6) comprend (comprennent) en outre un guide (16) pour l'élément résilient (9) et l'élément résilient (9) est monté dans le guide (16).

5. Implant (3) selon la revendication 4, dans lequel l'élément résilient (9) comprend une partie filetée (21) ou est raccordé à un autre élément (15) comprenant une partie filetée (21) et est monté dans le guide (16) au moyen d'au moins cette partie filetée (21), de sorte que l'élément résilient (9) puisse être étendu ou rétracté en faisant tourner cette partie (21).

6. Implant (3) selon la revendication 4 ou 5, dans lequel le guide (16) est au moins partiellement incurvé et pousse l'élément résilient (9) vers la structure osseuse (1).

7. Implant (3) selon l'une quelconque des revendications précédentes, dans lequel au moins la forme de la collerette (5) de l'implant est modelée pour s'adapter étroitement à la forme du bord (4) du manque (2), de sorte que, lors de la mise en place de l'implant (3) dans le manque (2), il ne reste qu'un degré de liberté.

8. Implant (3) selon l'une quelconque des revendications précédentes, dans lequel l'élément extensible (9) est suffisamment résilient pour compenser la réduction locale de l'épaisseur de l'os (11).
